# EUROPEAN PATENT SPECIFICATION

(11) **EP 2 017 330 B1**
(45) Date of publication and mention of the grant of the patent: **11.08.2010**
(21) Application number: 08158556.4
(22) Date of filing: 19.06.2008
(51) Int. Cl.: C12M 3/00

(54) **Microinjection apparatus and method of injecting fluid**
Mikroinjektionsvorrichtung und Verfahren zur Injektion eines Fluids
Appareil de micro-injection et procédé d'injection de fluide

(30) Priority: 18.07.2007 JP 2007187678
(43) Date of publication of application: 21.01.2009
(73) Proprietor: Fujitsu Limited, Kawasaki-shi, Kanagawa 211-8588 (JP)
(72) Inventor: NISHIYAMA, Shusaku c/o Fujitsu Limited, Kanagawa 211-8588 (JP)
(74) Representative: Wilding, Frances Ward

(56) References cited:
- JP-A- 3 035 160
- US-A1- 2007 053 792

## Description

### BACKGROUND OF THE INVENTION

### Field of the Invention:

An aspect of the present invention relates to a microinjection apparatus allowing the injection of a substance or fluid, such as medicine, into an object or minute body, such as a cell, for example.

### Description of the Prior Art:

Amicroinjection apparatus is well known. A suction pump is utilized to capture cells, dispersed in a solution within a Petri dish, on a capturing plate, for example, in the microinjection apparatus. A capillary is then driven to move from a withdrawn position to an injection position, so that the tip end of the capillary is stuck into a target cell among the captured cells. Fluid such as medicine is injected from the tip end of the capillary into the target cell, for example. The capillary thereafter withdraws to the withdrawn position. Subsequently, such an injection process is continuously applied to the other cells one by one.

When the capillary is driven to move from the injection position to the withdrawn position, for example, a cell or cells sometimes adhere to the tip end of the capillary. Such a cell or cells hinder the insertion of the tip end of the capillary into the next target cell. This results in interruption of the injection process of the medicine to the next target cell. The tip end of the capillary has a significantly small outer diameter in a range from 0.2 micrometers approximately to several micrometers. Even if a small amount of force is applied to the tip end of the capillary to remove the cell or cells, the tip end of the capillary possibly gets broken. Accordingly, if a cell or cells adhere to the tip end of the capillary, it inevitably spends a lot of time to remove the cell or cells. In addition, even when the continuous injection is successfully realized for the cells, adhesion such as fragments of the cell wall, the mucosa-like substance, or the like, increases at the outer wall of the capillary. Such adhesion promotes the adhesion of a cell or cells to the tip end of the capillary.

US-A-20070053792 describes a sampler comprising an air injection concentric tube adapted to clean the sampler tip.

### SUMMARY OF THE INVENTION

It is desirable to provide a microinjection apparatus and a method of injecting fluid, contributing to a continuous application of an injection process of fluid.

According to an embodiment of an aspect of the present invention, there is provided a microinjection apparatus comprising: a support member defining an inner space receiving a discharge pressure; a capillary connected to the support member, the capillary discharging a first fluid from the tip end of the capillary in response to the discharge pressure supplied from the inner space of the support member; and a pipe member connected to the capillary, the pipe member defining a flow passage for a second fluid directed toward the tip end of the capillary.

### BRIEF DESCRIPTION OF THE DRAWINGS

The above and other objects, features and advantages of embodiments of the present invention will become apparent from the following description of the preferred embodiment in conjunction with the accompanying drawings, wherein:
Fig. 1 is a schematic view of a microinjection apparatus according to an embodiment of the present invention;
Fig. 2 is a sectional view schematically illustrating a capillary unit according to a first specific example;
Fig. 3 is a block diagram showing a controlling system according to the embodiment;
Fig. 4 is a flowchart showing the procedure of an injection process of a medicine;
Fig. 5 is a schematic view illustrating an injection unit set at a withdrawn position;
Fig. 6 is a graph showing the relationship between the position of the capillary unit and the amount of a supplied fluid;
Fig. 7 is a schematic view illustrating the capillary unit advancing into a culture medium;
Fig. 8 is a schematic view illustrating the injection of medicine into a cell;
Fig. 9 is a schematic view illustrating the process for detecting a attachments;
Fig. 10 is a schematic view illustrating the process for removing the attachments;
Fig. 11 is a flowchart showing the procedure of a washing process;
Fig. 12 is a schematic view illustrating the injection unit set at a washing position; and
Fig. 13 is a sectional view illustrating a capillary unit according to a second specific example.

### DESCRIPTION OF THE PREFERRED EMBODIMENT

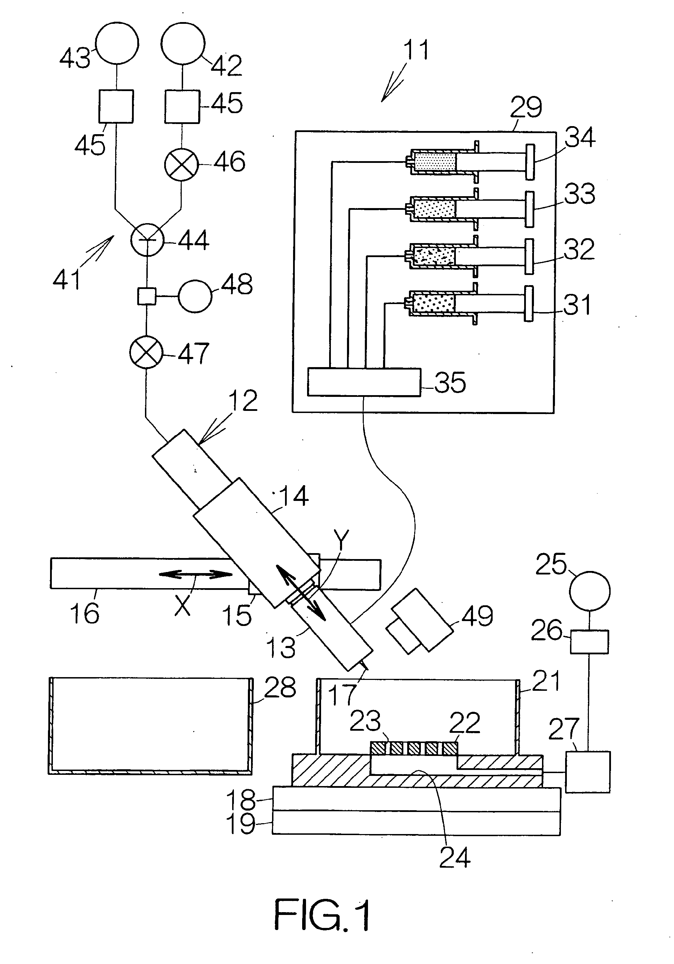

Fig. 1 schematically illustrates a microinjection apparatus 11 according to an embodiment of the present invention. The microinjection apparatus 11 includes an injection unit 12 for injecting medicine into a cell, for example. The injection unit 12 includes a support section, namely a support member 14, supporting a capillary unit 13. The capillary unit 13 is detachably attached to the tip end of the support member 14.

The support member 14 is attached to a guided block 15. The guided block 15 is attached to a guide rail 16 extending in a horizontal direction or the direction of an x-axis for a relative sliding movement. The support member 14 or injection unit 12 is in this manner allowed to slide along the x-axis. A first linear driving mechanism such as a linear motor is utilized to realize such a sliding movement, for example.

The support member 14 is attached to the guided block 15 for relative sliding movement in the direction of a y-axis. The support member 14 or injection unit 12 is in this manner allowed to slide along the y-axis intersecting, at a predetermined inclination angle, with a horizontal plane including the x-axis. A second linear driving mechanism such as a linear motor is utilized to realize such a sliding movement, for example.

The capillary unit 13 includes a capillary 17. The tip end of the capillary 17 is stuck into a cell for the injection of medicine into the cell, for example. The capillary 17 is designed to extend along the y-axis. The y-axis corresponds to the longitudinal axis of the injection unit 12 or capillary 17. The sliding movement of the support member 14 along the y-axis realizes forward and backward movements of the capillary unit 13 or capillary 17. A detailed description will be made on the capillary unit 13 later.

First and second work stages 18, 19 are opposed to the injection unit 12. The first work stage 18 is allowed to move in a first direction along a horizontal plane. The second work stage 19 is allowed to move in a second direction perpendicular to the first direction along a horizontal plane. A first horizontal driving mechanism such as a linear motor is incorporated in the first work stage 18, for example. A second horizontal driving mechanism such as a linear motor is incorporated in the second work stage 19, for example. A container or Petri dish 21 is placed on the horizontal surface of the first work stage 18. Suspension is poured in the Petri dish 21, for example. The horizontal movements of the first and second work stages 18, 19 allow the movement of the Petri dish 21 along the horizontal surface.

A silicon chip 22 is fixed in the Petri dish 21. Through bores 23 are formed in the silicon chip 22. The through bores 23 penetrate from the front surface to the back surface of the silicon chip 22. The through bores 23 are connected to a path 24 formed in the Petri dish 21. A vacuum pump 25 is connected to the path 24. The vacuum pump 25 is designed to generate negative pressure. Air or liquid is in this manner sucked from the path 24. A pressure adjusting valve 26 is connected between the path 24 and the vacuum pump 25. The pressure adjusting valve 26 serves to keep the negative pressure constant in the path 24. A tank 27 is also connected between the path 24 and the vacuum pump 25. Liquid or culture medium flowing from the Petri dish 21 is kept in the tank 27 as described later.

A container 28 is placed near the Petri dish 21 for keeping wastewater, for example. The capillary unit 13 is driven to move along the x-axis from a reference position or withdrawn position to a washing position defined at a position outward from the withdrawn position in the horizontal direction. The container 28 is aligned with the washing position of the capillary unit 13. When the capillary 17 is set at the withdrawn position, the capillary 17 is opposed to the Petri dish 21. The capillary unit 13 is then driven to move along the y-axis between the withdrawn position and an injection position defined forward from the withdrawn position. When the capillary 17 is set at the washing position, the capillary 17 is opposed to the container 28.

A fluid supplying mechanism 29 is connected to the capillary unit 13. The fluid supplying mechanism 29 includes four fluid pumps, namely first to fourth fluid pumps 31, 32, 33, 34. The first fluid pump 31 is designed to keep a culture medium for cells. The second fluid pump 32 is designed to keep a diluent or pure water. The third fluid pump 33 is designed to keep a neutralizer or weakly acidic aqueous solution. A fourth fluid pump 34 is designed to keep a resolvent or alkaline aqueous solution. A switching valve 35 is connected between the capillary unit 13 and the first to fourth fluid pumps 31-34. The switching valve 35 is utilized to switch over the first to fourth fluid pumps 31-34 for connection to the capillary unit 13. The fluid kept in each of the first to fourth fluid pumps 31-34 is in this manner supplied to the capillary unit 13.

A pressurizing section, namely a pressure controlling mechanism 41, is connected to the support member 14. The pressure controlling mechanism 41 includes first and second pumps 42, 43. The pressure of the first pump 42 is set within a range between zero kilopascal and several kilopascals approximately. The pressure of the second pump 43 is set at 1, 000kPa approximately. The first and second pumps 42, 43 are connected to a switching mechanism 44. The switching mechanism 44 is connected to the support member 14. Regulators 45 are connected between the first pump 42 and the switching mechanism 44 and between the second pump 43 and the switching mechanism 44, respectively. The regulators 45 are utilized to adjust the pressure of the first and second pumps 42, 43. The action of the switching mechanism 44 enables establishment of a connection between the capillary 17 and one of the first and second pumps 42, 43.

A first valve 46 is connected between the first pump 42 andtheswitchingmechanism44. The opening/closing of the first valve 46 allows connection/disconnection between the first pump 42 and the switching mechanism 44. A second valve 47 is connected between the switching mechanism 44 and the capillary 17. The opening/closing of the second valve 47 allows connection/disconnection between the switching mechanism 44 and the capillary 17. A pressure sensor 48 is connected between the switching mechanism 44 and the second valve 47. The pressure sensor 48 is designed to detect the pressure within the capillary 17.

An image capturing apparatus 49 is placed near the injection unit 12. A camera is employed as the image capturing apparatus 49. When the injection unit 12, namely the capillary unit 13, is set at the withdrawn position, the image capturing apparatus 49 is opposed to the tip end of the capillary 17. The image capturing apparatus 49 sets the optical axis of the lens in the direction perpendicular to the longitudinal axis of the capillary 17, for example. The image capturing apparatus 49 is in this manner utilized to capture an image of the tip end of the capillary 17.

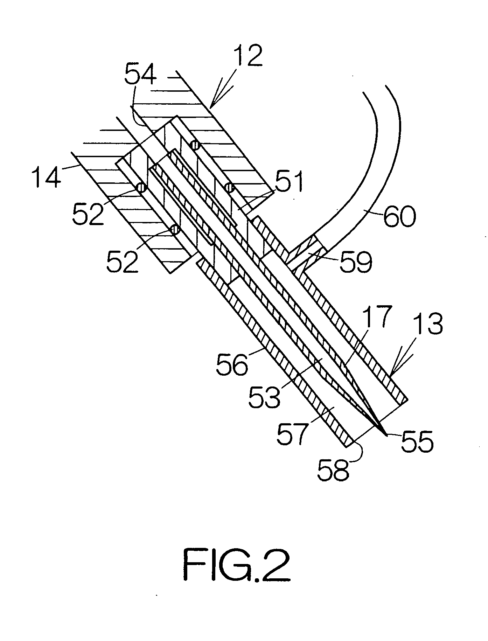

Fig. 2 schematically illustrates the capillary unit 13. As shown in Fig. 2, the capillary unit 13 includes a cylindrical unit body 51, for example. The unit body 51 is placed in a columnar space defined in the tip end of the support member 14. The unit body 51 is made of a resin material such as plastic, for example. Annular rings 52 are placed within the columnar space of the support member 14. The annular rings 52 are made of a silicone rubber, for example. When the unit body 51 is inserted in the annular rings 52, the annular rings 52 elastically deform. The unit body 51 or capillary unit 13 is thus removably supported on the support member 14.

The root end of the capillary 17 is received in the unit body 51. An adhesive is utilized to fix the capillary 17 to the unit body 51. The capillary 17 is made out of a cylindrical glass. The capillary 17 defines a flow passage 53 designed to extend along the longitudinal axis of the capillary 17. The rear end of the flow passage 53 is connected to an inner space 54 defined in the support member 14. The inner space 54 is connected to the aforementioned pressure controlling mechanism 41. The capillary 17 is formed in the shape tapered toward its tip end. The capillary 17 defines a minute hole 55 for injection at its tip end. The tip end of the flow passage 53 opens at the minute hole 55. The outer diameter of the capillary 17 is set equal to or smaller than 1.0mm approximately, for example.

An adhesive is utilized to fix a cylindrical outer sheath, namely a pipe member 56, to the unit body 51. The unit body 51 is fitted into the pipe member 56. The pipe member 56 is made of a resin material such as plastic, for example. The capillary 17 is placed within a columnar space defined in the pipe member 56. The pipe member 56 surrounds the capillary 17 around the longitudinal axis of the capillary 17. The pipe member 56 is set coaxial with the capillary 17. A constant space is thus established between the inner periphery of the pipe member 56 and the outer periphery of the capillary 17 in the centrifugal direction from the longitudinal axis of the capillary 17. The tip end of the capillary 17 protrudes forward from the tip end of the pipe member 56. The outer diameter of the pipe member 56 is set at 3.0mm approximately, for example.

A flow passage 57 is defined between the inner periphery of the pipe member 56 and the outer periphery of the capillary 17. The flow passage 57 is designed to extend in parallel with the flow passage 53 of the capillary 17. The rear end of the flow passage 57 is closed with the front end surface of the unit body 51. The front end of the flow passage 57 is open. An outlet 58 is defined between the front end of the pipe member 56 and the outer periphery of the capillary 17. The flow passage 57 opens at the outlet 58. The tip end of the capillary 17 protrudes from the outlet 58. The flow passage 57 thus extends from the root end to the tip end of the capillary 17. The pipe member 56 defines a connection opening 59 at a position adjacent to the rear end of the flow passage 57. The aforementioned fluid supplying mechanism 29 is connected to the connection opening 59 through a supplying passage 60. The supplying passage 60 may be made of a tube, for example.

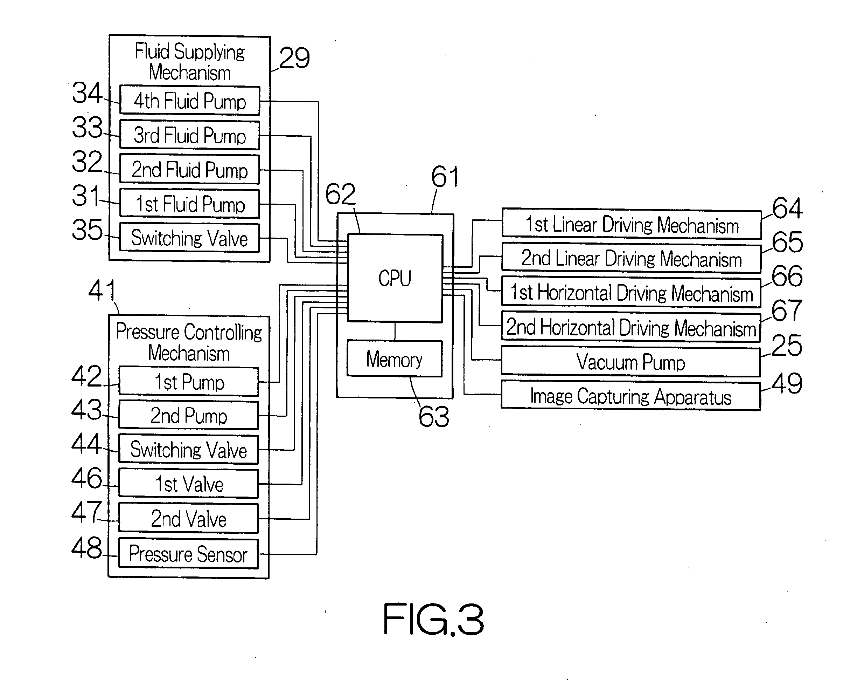

As shown in Fig. 3, the microinjection apparatus 11 includes a computer 61. The computer 61 includes electronic circuit elements such as a central processing unit (CPU) 62 and a memory 63. The CPU 62 is designed to execute a various kinds of processing based on a software program and data temporarily stored in the memory 63, for example. The software program includes a software program for controlling the microinjection apparatus 11 according to the present embodiment. The software program and data may be stored in a mass storage device such as a hard disk drive (HDD) likewise contained within the computer 61.

The CPU 62 is connected to the aforementioned first linear driving mechanism 64, the second linear driving mechanism 65, the first horizontal driving mechanism 66 and the second horizontal driving mechanism 67. The CPU 62 outputs driving signals for controlling the first and second linear driving mechanisms 64, 65 and the first and second horizontal driving mechanisms 66, 67. The CPU 62 is also connected to the vacuum pump 25. The CPU 62 outputs a controlling signal for controlling the vacuum pump 25.

The CPU 62 is connected to the aforementioned fluid supplying mechanism 29 and the pressure controlling mechanism 41. The CPU 62 outputs controlling signals for switching over the connection of the switching valve 35. The CPU 62 also outputs a controlling signal for driving one of the first to fourth fluid pumps 31-34. The fluid is thus supplied to the flow passage 57 through the supplying passage 60. The CPU 62 also outputs controlling signals for controlling the first and second pumps 42, 43, the first and second valves 46, 47 and the switching mechanism 44. The pressure controlling mechanism 41 is in this manner allowed to apply pressure to the columnar space of the support member 14, namely to the flow passage 53 of the capillary 17. A detection signal from the pressure sensor 48 is referred to for controlling the pressure controlling mechanism 41.

The CPU 62 is connected to the aforementioned image capturing apparatus 49. When the capillary 17 is set at the withdrawn position, the CPU 62 outputs a controlling signal to the image capturing apparatus 49 to capture the image of the tip end of the capillary 17 with the image capturing apparatus 49. The captured image is output to the CPU 62. The captured image is utilized to detect whether or not an undesirable attachment adheres to the tip end of the capillary 17 as described later. A description will be made on such a detection process later in detail. The CPU 62 is designed to count the shots of capturing the image.

An attachment or attachments such as fragments of the cell wall, cytoplasm, mucosa, and the like, possibly adhere to the tip end of the capillary 17. A washing process is applied to the capillary 17 for removal of the attachment or attachments. Apredetermined threshold is set in the computer 61 for the washing process. The threshold is stored in the memory 63. The threshold is specified based on an interval of adhesion to the tip end of the capillary 17, for example. The interval of adhesion is specified as the number of the injection of medicine during a period of time between the last adhesion and the subsequent adhesion. The image capturing apparatus 49 is programmed to capture the image of the tip end of the capillary 17 every time when an injection process is applied to a cell. The number of the captured images is thus equal to the number of the injection applied. Accordingly, the number of shots of capturing the image is utilized to count the number of the injection applied. Here, the threshold of the interval of adhesion is determined based on experience, for example. The interval of adhesion may be set within a range between 100 times to 200 times approximately, for example. A description will be made on the washing process later in detail.

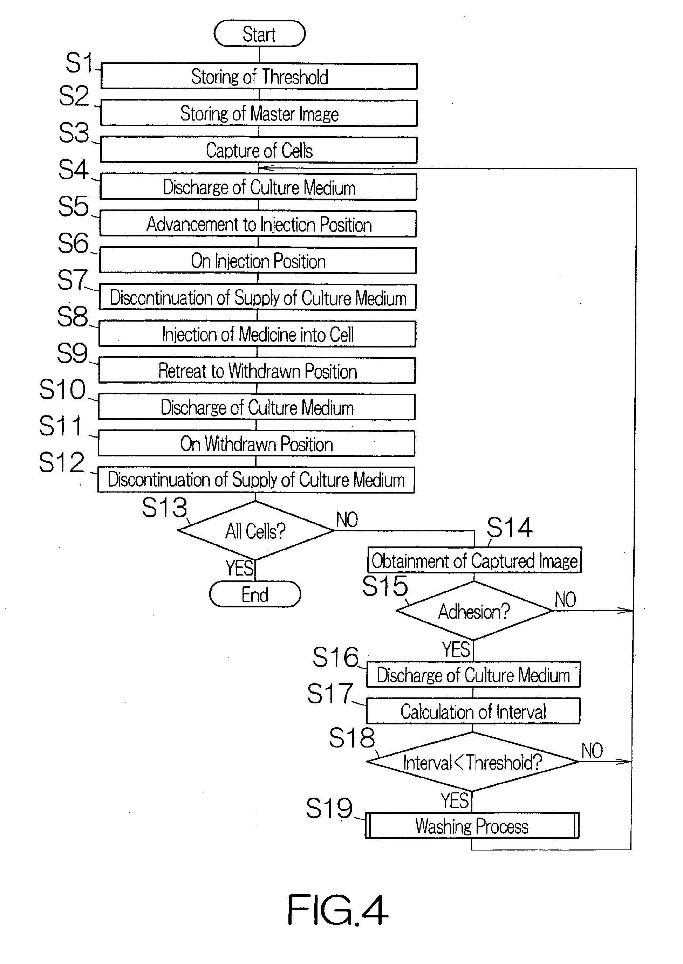

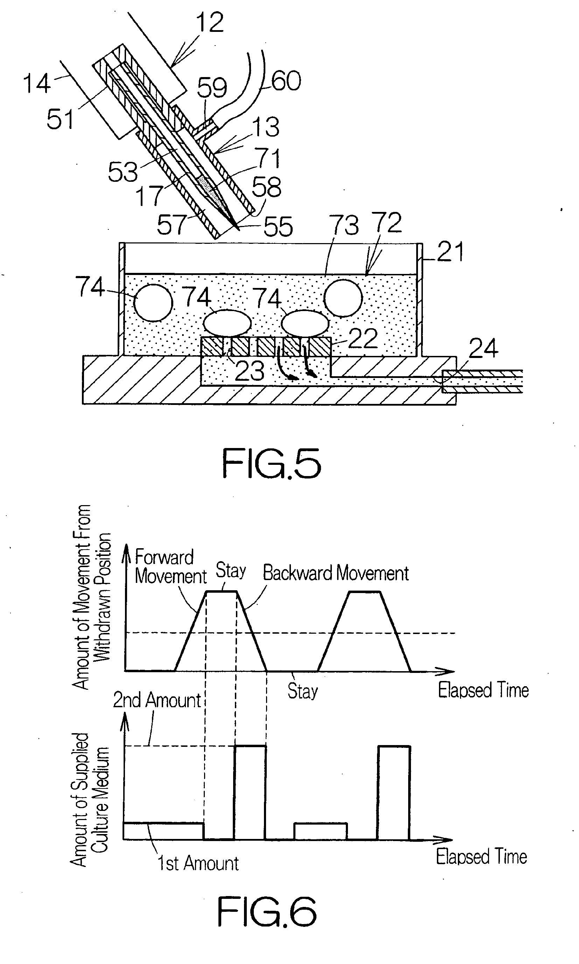

Now, assume that medicine is to be injected into a cell. The threshold of the aforementioned interval of adhesion is first input into the computer 61. At step S1 in Fig. 4, the CPU 62 operates to store the threshold in the memory 63. The support member 14 has been set at the withdrawn position. The capillary unit 13 has been attached to the tip end of the support member 14. As shown in Fig. 5, the capillary 17 has been filled with a predetermined amount of medicine 71. The fluid supplying mechanism 29 is connected to the connection opening 59 of the pipe member 56 through the supplying passage 60. The CPU 62 outputs a controlling signal to the image capturing apparatus 49 for capturing the image of the tip end of the capillary 17 in this state. The captured image is output to the CPU 62. At step S2, the captured image is stored in the memory 63 as a master image based on the instructions from the CPU 62.

The Petri dish 21 is set on the horizontal surface of the first work stage 18. Suspension 72 is dropped onto the Petri dish 21. The suspension 72 contains a culture medium 73 and cells 74 dispersed in the culture medium 73. The CPU 62 outputs a controlling signal to drive the vacuum pump 25. The action of the vacuum pump 25 allows generation of negative pressure in the path 24. The pressure adjusting valve 26 serves to keep the negative pressure constant. The generated negative pressure serves to induce a flow of the culture medium 73 into the path 24 through the bores 23 from the Petri dish 21. The culture medium 73 then flows into the tank 27. Since the culture medium 73 is kept in the tank 27, the pressure adjusting valve 26 and the vacuum pump 25 are reliably prevented from receiving the culture medium 73. At step S3, the cells 74 dispersed in the culture medium 73 are captured at predetermined positions, namely on the openings of the through bores 23, based on suction. The CPU 62 operates to position the first and second work stages 18, 19 at a predetermined reference position.

At step S4, the CPU 62 then operates to switch the switching valve 35. The first fluid pump 31 is selected at step S4. When the switching valve 35 is switched, culture medium is supplied to the capillary unit 13 from the first fluid pump 31. Here, the culture medium is the same as the culture medium 73 in the Petri dish 21, for example. Fig. 6 is a graph showing the relationship between the amount of movement and the amount of the supplied culture medium. The amount of movement corresponds to the amount of the movement of the capillary unit 13 in the direction of the y-axis. The amount of the supplied culture medium corresponds to the amount of the culture medium supplied to the capillary unit 13. As shown in Fig. 6, the amount of the supplied culture medium is kept at a first amount until the capillary unit 13 reaches the injection position through a forward movement. The supply of the culture medium to the capillary unit 13 serves to generate a flow of the culture medium within the flow passage 57 of the pipe member 56 toward its tip end. The culture medium is discharged from the outlet 58 in parallel with the longitudinal axis of the capillary 17. The CPU 62 simultaneously operates to drive the second linear driving mechanism 65 at step S5. The action of the second linear driving mechanism 65 allows the forward movement of the capillary unit 13 from the withdrawn position to the injection position.

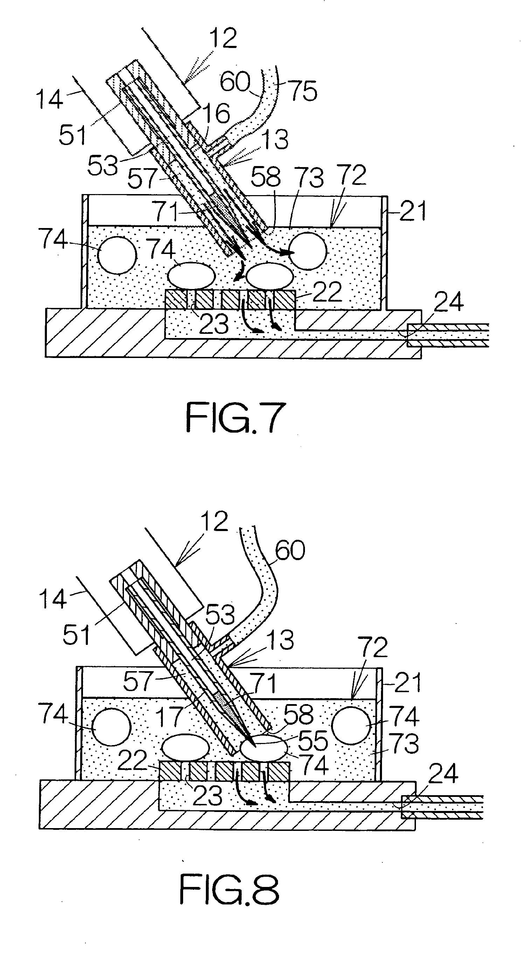

The tip end of the capillary unit 13 advances in the culture medium 73. As shown in Fig. 7, for example, culture medium 75 is kept discharged from the outlet 58 toward the tip end of the capillary 17 during the advancement. A flow of the culturemedium 75 serves to push away the cells 74 floating around the tip end of the capillary 17. The capillary 17 is thus reliably prevented from an accidental insertion into the cells 74 floating in the culture medium 73. At step S6, when the injection unit 12 reaches the injection position, the tip end of the capillary 17 is inserted into the cell 74 held at a predetermined position, as shown in Fig. 8. At step S7, the CPU 62 operates to discontinue the supply of the culture medium 75 from the first fluid pump 31. The discharge of the culture medium 75 from the outlet 28 is thus stopped.

In this case, the CPU 62 operates to keep the first and second valves 46, 47 in the closed state. The CPU 62 outputs a controlling signal to the switching mechanism 44 to switch over the connection of the first pump 42 to the connection of the second pump 43. A predetermined discharge pressure is correspondingly set in a space between the second pump 43 and the second valve 47. The CPU 62 refers to the pressure value of the pressure sensor 48 in setting of the discharge pressure. When the predetermined discharge pressure has been set, the CPU 62 outputs a controlling signal to the switching mechanism 44 to switch over the connection of the second pump 43 to the connection of the first pump 42. When the tip end of the capillary 17 has stuck into the cell 74, the CPU 62 operates to open the second valve 47. The discharge pressure acts on the root end of the flow passage 53, namely the rear end of the capillary 17. The flow passage 53 thus receives the discharge pressure. The discharge pressure serves to allow the medicine 71 within the flow passage 53 to flow out of the minute hole 55. The medicine 71 of a predetermined amount is in this manner injected into the cell 74 through the minute hole 55 of the capillary 17 at step S8.

When the injection of the medicine 71 has been completed, the CPU 62 operates to open the first valve 46. A regular pressure thus acts on the capillary 17. A pressure of a predetermined value is maintained within the capillary 17. The capillary 17 stops the discharge of the medicine 71 through the minute hole 55. The CPU 62 operates to close the first and second valves 46, 47. At step S9, the CPU 62 operates to drive the second linear driving mechanism 65. The capillary unit 13 is thus allowed to move backward from the injection position to the withdrawn position. At step S10, the CPU 62 operates to drive the first fluid pump 31 during the backward movement of the capillary unit 13. The action of the first fluid pump 31 allows the supply of the culture medium 75 to the capillary unit 13. As is apparent from Fig. 6, the amount of the supplied culture medium 75 is set at a second amount, larger than the first amount, during the backward movement of the capillary unit 13. When the tip end of the capillary 17 is withdrawn out of the cell 74, the culture medium 75 is discharged from the outlet 58 toward the cell 74. The flow of the culture medium 75 serves to drive the cell 74 away. The tip end of the capillary 17 is reliably prevented from attachment of the cell 74 during the backward movement of the capillary unit 13 to the withdrawn position. When the injection unit 12 has reached the withdrawn position at step S11, the CPU 62 operates to stop the discharge of the culture medium 75 from the first fluid pump 31.

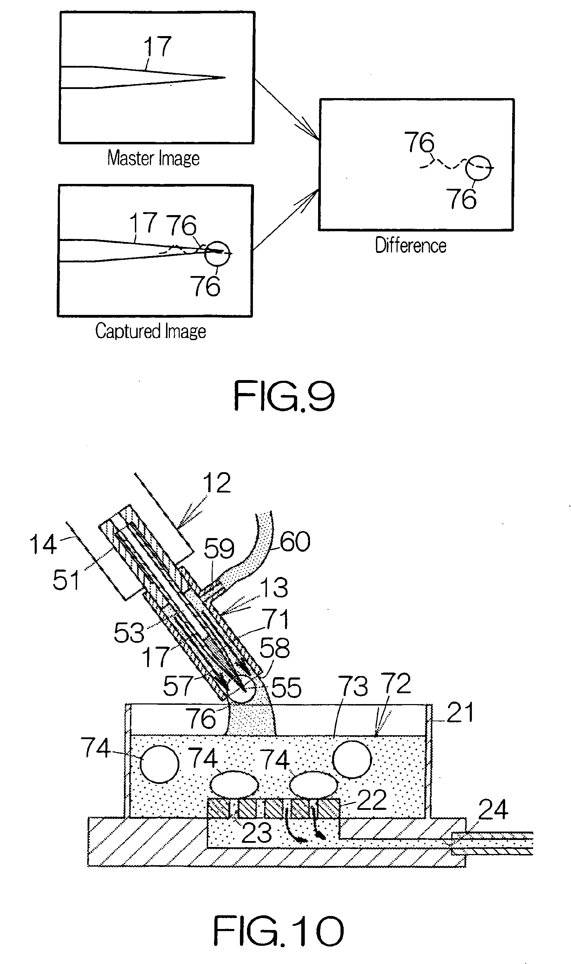

The CPU 62 then judges whether or not the medicine 71 has been injected into all the cells 74 at step S13. If the injection of the medicine 71 has not yet been completed, the CPU 62 outputs a controlling signal to the image capturing apparatus 49 for capturing the image of the tip end of the capillary 17. The image of the tip end of the capillary 17 is captured every time when the injection has been finished for a cell. The captured image is output to the CPU 62. The CPU 62 operates to compare the captured image with the master image stored in the memory 63. A difference is observed between the captured image and the master image. Fig. 9 illustrates the observation of a difference between the captured image and the master image. If an undesirable attachment does not adhere to the tip end of the capillary 17, there is no difference between the captured image and the master image. Accordingly, when a difference is not observed, the CPU 62 determines no detection of any attachment adhering to the tip end of the capillary 17. The processing of the CPU 62 returns to step S4. The injection of the medicine 71 for one of the cells 74 is in this manner completed. Subsequently, the injection is applied to another cell 74. The CPU 62 operates to drive the first and second horizontal driving mechanisms 66, 67. The Petri dish 21 is thus allowed to move in the horizontal direction. The injection of the medicine 71 is in this manner continuously applied to the cells 74 one by one. When the medicine 71 is injected into all the cells 74, the processing of the CPU 62 is completed.

If a difference is observed between the captured image and the master image, as shown in Fig. 9, for example, at step S15, the CPU 62 determines the existence of undesirable attachments 76 adhering to the tip end of the capillary 17. The master image includes no image of an attachment adhering to the tip end of the capillary 17. The captured image includes an image of the undesirable attachments 76 adhering to the tip end of the capillary 17. Accordingly, the subtraction of the master image from the captured image results in obtainment of the attachments 76. In this case, the processing proceeds to step S16. At step S16, the CPU 62 operates to drive the first fluid pump 31. The action of the first fluid pump 31 generates a flow of the culture medium 75 within the flow passage 57 of the pipe member 56 toward the tip end of the flow passage 57. The culture medium 75 is discharged from the outlet 58. As shown in Fig. 10, for example, the flow of the culture medium 75 generates a shearing force or slippage stress on the attachments 76 along the longitudinal axis of the capillary 17. The slippage stress allows removal of the attachments 76 from the tip end of the capillary 17. Since the tip end of the capillary 17 at the withdrawn position is located above the Petri dish 21, the attachments 76 and the culture medium 75 are discharged from the outlet 58 into the Petri dish 21.

At step S17, the CPU 62 refers to the interval of adhesion. The interval of adhesion corresponds to the number of the injection of the medicine 71 between the continuous or sequential adhesions. Specifically, the CPU 62 refers to the number of the shots capturing the image. At step S18, the detected interval of adhesion is compared with the threshold stored in the memory 63. If the attachments 76 adhere at an interval shorter than the threshold, the CPU 62 determines establishment of conditions liable to suffer from adhesion of the attachments 76 to the tip end of the capillary 17. Specifically, if the detected interval of adhesion is equal to or larger than the threshold, the processing of the CPU 62 returns to step S4. The injection is thus continuously applied to the cells 74 one by one. If the detected interval of adhesion is smaller than the threshold, the CPU 62 determines establishment of conditions liable to suffer from adhesion of the attachments 76 to the tip end of the capillary 17. The processing of the CPU 62 then proceeds to step S19. At step S19, the CPU 62 operates to subject the capillary 17 to a washing process. The CPU 62 simultaneously sets the interval of adhesion or the number of shots capturing the image to zero.

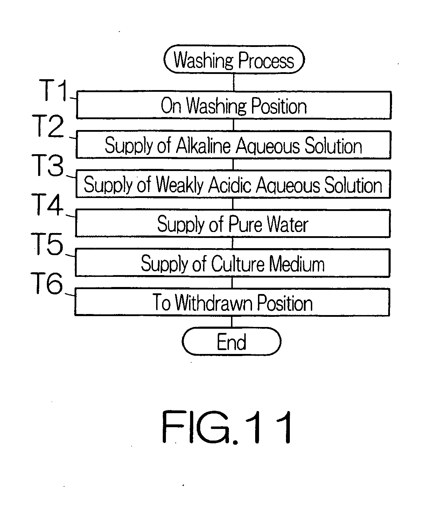

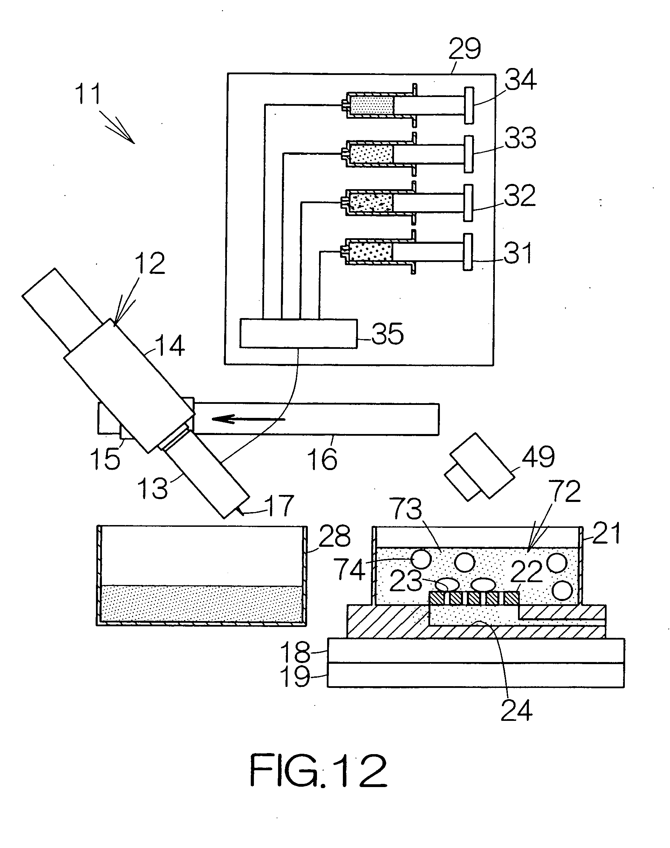

Fig. 11 is a flowchart showing the detailed procedure of the washing process of step S19 shown in Fig. 4. At step T1, the CPU 62 operates to drive the first linear driving mechanism 64. The action of the first linear driving mechanism 64 allows the movement of the capillary unit 13 from the withdrawn position to the washing position. The capillary 17 at the washing position locates the tip end of the capillary 17 above the container 28, as shown in Fig. 12. The CPU 62 operates to switch over the switching valve 35 for the connection to the fourth fluid pump 34 at step T2. Alkaline aqueous solution of a predetermined amount is supplied from the fourth fluid pump 34 to the flow passage 57 of the pipe member 56. The alkaline aqueous solution is discharged from the outlet 58 toward the tip end of the capillary 17. The discharged alkaline aqueous solution serves to dissolve the attachments 76 adhering to the tip end of the capillary 17. The attachments 76 are removed from the tip end of the capillary 17.

The CPU 62 then operates to switch over the switching valve 35 for the connection to the third fluid pump 33 at step T3. Weakly acidic aqueous solution of a predetermined amount is supplied from the third fluid pump 33 to the flow passage 57. The alkaline aqueous solution can possibly remain at the tip end of the capillary 17. The remaining alkaline aqueous solution can interfere with the injection process of the medicine 71 to the cell. When the weakly acidic aqueous solution is supplied to the flow passage 57, a chemical reaction occurs between the weakly acidic aqueous solution and the alkaline aqueous solution. The alkaline aqueous solution is thus neutralized at the tip end of the capillary 17. The CPU 62 then operates to switch over the switching valve 35 for the connection to the second fluid pump 32 at step T4. Pure water of a predetermined amount is supplied from the second fluid pump 32 to the flow passage 57. The supply of the pure water serves to dilute and remove deposition resulting from the neutralization between the alkaline aqueous solution and the weakly acidic aqueous solution. Likewise, the supply of the pure water serves to dilute and remove residue of the weakly acidic aqueous solution.

The CPU 62 then operates to switch over the switching valve 35 for the connection to the first fluid pump 31 at step T5. The first fluid pump 31 operates to supply culture medium to the flow passage 57. The attachments 76 are in this manner washed away from the tip end of the capillary 17. The supplied culture medium is kept in the flow passage 57. Since the capillary 17 is positioned above the container 28, the container 28 reliably receives wastewater such as the alkaline aqueous solution, the weakly acidic aqueous solution, the pure water and the culture medium, discharged from the outlet 58. The washing process of the capillary 17 is in this manner completed. The CPU 62 then operates to drive the first linear driving mechanism 64 at step T6. The action of the first linear driving mechanism 64 allows the injection unit 12 to return to the withdrawn position from the washing position. The processing of the CPU 62 then returns to step S4. The injection of the medicine 71 is continuously applied to the other cells 74.

The microinjection apparatus 11 allows the capillary unit 13 to receive the supply of the culture medium 75 when the capillary 17 moves forward from the withdrawn position to the injection position. The culture medium 75 is discharged from the outlet 58 toward the tip end of the capillary 17 along the outer periphery of the capillary 17. The discharged culture medium 75 serves to drive away the cells 74 floating in the culture medium 73 at a position adjacent to the tip end of the capillary 17. The capillary 17 is thus prevented from suffering from an accidental insertion into the cells 74. The mentioned action of removing the floating cells 74 does not interrupt the injection of the medicine 71 to the cell 74. The injection of the medicine 71 is continuously applied to the cells 74 with efficiency.

Likewise, when the capillary 17 moves backward from the injection position to the withdrawn position, the culture medium 75 is supplied to the capillary unit 13. The culture medium 75 is discharged from the outlet 58 toward the cell 74 at the tip end of the capillary 17 along the outer surface of the capillary 17. Even if the tip end of the capillary 17 suffers from adhesion of the cell or cells 74 during the backward movement of the capillary 17, the discharged culture medium 75 serves to drive the cell or cells 74 away. Accordingly, the capillary 17 is reliably prevented from adhesion of the cell or cells 74. The mentioned action of removing the cell or cells 74 does not interrupt the injection of the medicine 71 to the cell 74. The injection of the medicine 71 is thus continuously applied to the cells 74 with efficiency.

In addition, even if the attachments 76, such as fragments of cell wall, cytoplasm, mucosa, and the like, adheres to the tip end of the capillary 17, the flow of the culture medium 75 discharged from the outlet 58 generates a slippage stress on the attachments 76 along the longitudinal axis of the capillary 17. The attachments 76 are thus removed from the tip end of the capillary 17. The capillary 17 is in this manner prevented from suffering from adhesion of the attachments 76. The avoidance of the adhesion of the cells 74 to the capillary 17 enables a continuous application of the injection of the medicine 71 to the cells 74 with efficiency.

Moreover, when the attachments 76 are detected at the tip end of the capillary 17, the tip end of the capillary 17 is subjected to the washing process. The attachments 76, such as fragments of cell wall, cytoplasm, mucosa, and the like, are dissolved in the alkaline aqueous solution. The outer surface of the capillary 17 is then subjected to the neutralization with the weakly acidic aqueous solution and the pure water. The washing process is applied in this manner. The attachments 76 are thus removed from the tip end of the capillary 17. The washed outer surface of the capillary 17 promotes prevention of adhesion of attachments 76 to the capillary 17. This prevention of adhesion enables a continuous application of the injection of the medicine 71 to the cells 74 with efficiency.

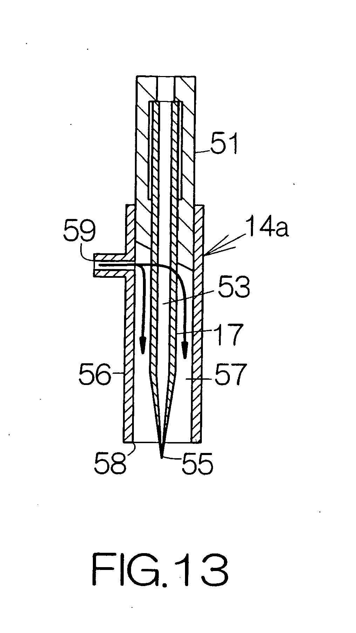

It should be noted that the capillary unit 13 may be replaced with new one in the case where the medicine 71 in the capillary 17 is used up. A capillary unit 13a may be utilized in place of the aforementioned capillary unit 13, as shown in Fig. 13. The capillary unit 13a includes the unit body 51 defining the front end surface extending along an imaginaryplane intersecting with the longitudinal axis of the capillary 17 at a predetermined inclination angle. The front end of the unit body 51 is opposed to the connection opening 59 of the pipe member 56. Like reference numerals are attached to the structure or components equivalent to those of the aforementioned capillary unit 13. The front end surface of the unit body 51 serves to direct the flow of a fluid, running through the connection opening 59 into the flow passage 57, in the direction in parallel with the longitudinal axis of the capillary 17. The flow of the fluid can be established from the outlet 58 toward the tip end of the capillary 17 in a facilitated manner.

## Claims

1. A microinjection apparatus comprising:
a support member defining an inner space receiving a discharge pressure;
a capillary connected to the support member, the capillary discharging a first fluid from a tip end of the capillary in response to the discharge pressure supplied from the inner space of the support member; and
a pipe member connected to the capillary, the pipe member defining a flow passage for a second fluid directed toward the tip end of the capillary.

2. The microinjection apparatus according to claim 1, wherein the flow passage extends from a root end of the capillary to the tip end of the capillary in parallel with a longitudinal axis of the capillary.

3. The microinjection apparatus according to claim 1 or 2, wherein the capillary is placed within an inner space defined in the pipe member, the capillary having the tip end protruding from an outlet defined at one end of the pipe member.

4. The microinjection apparatus according to any preceding claim, wherein the pipe member is formed coaxially with the capillary.

5. Themicroinjection apparatus according to any preceding claim, further comprising:
a driving mechanism allowing a backward movement of the capillary along the longitudinal axis of the capillary; and
a fluid supplying mechanism allowing supply of the second fluid to the flow passage during the backward movement of the capillary.

6. The microinjection apparatus according to claim 5, wherein the fluid supplying mechanism allows the supply of the second fluid to the flow passage during a forward movement of the capillary.

7. The microinjection apparatus according to any preceding claim, further comprising:
a driving mechanism allowing a forward movement of the capillary along a longitudinal axis of the capillary; and
a fluid supplying mechanism allowing supply of the second fluid to the flow passage during the forward movement of the capillary.

8. The microinjection apparatus according to any preceding claim, further comprising:
an image capturing apparatus utilized to capture an image of the tip end of the capillary; and
a fluid supplying mechanism allowing supply of the second fluid to the flow passage when an adhesion of a substance is detected based on the image captured using the image capturing apparatus.

9. The microinjection apparatus according to claim 8, further comprising:
a container connected to the pipe member to keep a dissolvent for dissolving the substance; and
a container connected to the pipe member to keep a neutralizer for neutralizing the dissolvent.

10. A method of injecting a fluid, comprising:
injecting a first fluid from a tip end of a capillary into an object captured in a solution at a predetermined position;
causing the tip end of the capillary to retreat after the first fluid has been injected; and
discharging a second fluid from an outlet when the capillary retreats, the second fluid directed toward the tip end of the capillary.

11. The method according to claim 10, further comprising:
moving the tip end of the capillary forward to the object; and
discharging the second fluid from the outlet when the capillary moves forward, the second fluid directed toward the tip end of the capillary.

12. The method according to claim 10 or 11, further comprising:
detecting whether or not an attachment adheres to the tip end of the capillary;
setting the tip end of the capillary at a position opposed to a predetermined container when adhesion of the attachment has been detected;
discharging a dissolvent from the outlet toward the tip end of the capillary for dissolving the attachment; and
discharging a neutralizer from the outlet toward the tip end of the capillary for neutralizing the dissolvent.

13. A method of making a minute body, comprising:
moving a capillary forward toward a minute body for injecting a substance into the minute body, a fluid being discharged from a position adjacent to the capillary toward the minute body;
stopping discharge of the fluid when the capillary is stuck into the minute body;
injecting the substance into the minute body; and
moving the capillary backward from the minute body, the fluid being discharged from a position adjacent to the capillary toward the minute body.

14. The method according to claim 13, wherein an image of the capillary is captured when the substance is injected into the minute body, the imaged captured being utilized to detect whether or not a substance adheres to the capillary.

15. The method according to claim 13 or 14, wherein a washing process is applied to the capillary if it is detected that a substance adheres to the capillary.

## Patentansprüche

1. Mikroinjektionsvorrichtung mit:
einem Stützglied, das einen Innenraum definiert, der einen Entladungsdruck empfängt;
einer Kapillare, die mit dem Stützglied verbunden ist, welche Kapillare ein erstes Fluid vom Ende einer Spitze der Kapillare als Reaktion auf den von dem Innenraum des Stützgliedes zugeführten Entladungsdruck entlädt; und
einem Röhrenglied, das mit der Kapillare verbunden ist, welches Röhrenglied einen Flussdurchgang für ein zweites Fluid definiert, das hin zu dem Ende der Spitze der Kapillare gelenkt wird.

2. Mikroinjektionsvorrichtung nach Anspruch 1, bei der sich der Flussdurchgang vom Ende einer Wurzel der Kapillare zum Ende der Spitze der Kapillare parallel zu einer Längsachse der Kapillare erstreckt.

3. Mikroinjektionsvorrichtung nach Anspruch 1 oder 2, bei der die Kapillare innerhalb eines in dem Röhrenglied definierten Innenraums angeordnet ist, wobei das Ende der Spitze der Kapillare aus einer Auslassöffnung herausragt, die an einem Ende des Röhrengliedes definiert ist.

4. Mikroinjektionsvorrichtung nach einem vorhergehenden Anspruch, bei der das Röhrenglied koaxial mit der Kapillare gebildet ist.

5. Mikroinjektionsvorrichtung nach einem vorhergehenden Anspruch, ferner mit:
einem Antriebsmechanismus, der eine Rückwärtsbewegung der Kapillare entlang der Längsachse der Kapillare zulässt; und
einem Fluidzuführungsmechanismus, der die Zuführung des zweiten Fluids zu dem Flussdurchgang während der Rückwärtsbewegung der Kapillare zulässt.

6. Mikroinjektionsvorrichtung nach Anspruch 5, bei der der Fluidzuführungsmechanismus die Zuführung des zweiten Fluids zu dem Flussdurchgang während einer vorwärtsbewegung der Kapillare zulässt.

7. Mikroinjektionsvorrichtung nach einem vorhergehenden Anspruch, ferner mit:
einem Antriebsmechanismus, der eine Vorwärtsbewegung der Kapillare entlang einer Längsachse der Kapillare zulässt; und
einem Fluidzuführungsmechanismus, der die Zuführung des zweiten Fluids zu dem Flussdurchgang während der Vorwärtsbewegung der Kapillare zulässt.

8. Mikroinjektionsvorrichtung nach einem vorhergehenden Anspruch, ferner mit:
einer Bilderfassungsvorrichtung, die genutzt wird, um ein Bild des Endes der Spitze der Kapillare zu erfassen; und
einem Fluidzuführungsmechanismus, der die Zuführung des zweiten Fluids zu dem Flussdurchgang zulässt, wenn ein Anhaften einer Substanz auf der Basis des unter Verwendung der Bilderfassungsvorrichtung erfassten Bildes detektiert wird.

9. Mikroinjektionsvorrichtung nach Anspruch 8, ferner mit:
einem Behälter, der mit dem Röhrenglied verbunden ist, um ein Lösungsmittel zum Lösen der Substanz aufzubewahren; und
einem Behälter, der mit dem Röhrenglied verbunden ist, um ein Neutralisationsmittel zum Neutralisieren des Lösungmittels aufzubewahren.

10. Verfahren zur Injektion eines Fluids, mit:
Injizieren eines ersten Fluids vom Ende einer Spitze einer Kapillare in ein Objekt, das in einer Lösung an einer vorbestimmten Position erfasst wird;
Bewirken des Zurückziehens des Endes der Spitze der Kapillare, nachdem das erste Fluid injiziert worden ist; und
Entladen eines zweiten Fluids von einer Auslassöffnung, wenn sich die Kapillare zurückzieht, welches zweite Fluid hin zu dem Ende der Spitze der Kapillare gelenkt wird.

11. Verfahren nach Anspruch 10, ferner mit:
Vorwärtsbewegen des Endes der Spitze der Kapillare zu dem Objekt; und
Entladen des zweiten Fluids von der Auslassöffnung, wenn sich die Kapillare vorwärts bewegt, welches zweite Fluid hin zu dem Ende der Spitze der Kapillare gelenkt wird.

12. Verfahren nach Anspruch 10 oder 11, ferner mit:
Detektieren, ob eine Anlagerung an dem Ende der Spitze der Kapillare haftet oder nicht;
Anordnen des Endes der Spitze der Kapillare an einer Position, die einem vorbestimmten Behälter gegenüberliegt, wenn das Anhaften der Anlagerung detektiert worden ist;
Entladen eines Lösungsmittels von der Auslassöffnung hin zu dem Ende der Spitze der Kapillare zum Lösen der Anlagerung; und
Entladen eines Neutralisationsmittels von der Auslassöffnung hin zu dem Ende der Spitze der Kapillare zum Neutralisieren des Lösungsmittels.

13. Verfahren zum Erzeugen eines sehr kleinen Körpers, mit:
Vorwärtsbewegen einer Kapillare hin zu einem sehr kleinen Körper zum Injizieren einer Substanz in den sehr kleinen Körper, wobei ein Fluid von einer an die Kapillare angrenzenden Position hin zu dem sehr kleinen Körper entladen wird;
Stoppen des Entladens des Fluids, wenn die Kapillare in dem sehr kleinen Körper steckt;
Injizieren der Substanz in den sehr kleinen Körper; und
Zurückbewegen der Kapillare von dem sehr kleinen Körper, wobei das Fluid von einer an die Kapillare angrenzenden Position hin zu dem sehr kleinen Körper entladen wird.

14. Verfahren nach Anspruch 13, bei dem ein Bild der Kapillare erfasst wird, wenn die Substanz in den sehr kleinen Körper injiziert wird, welches erfasste Bild genutzt wird, um zu detektieren, ob eine Substanz an der Kapillare haftet oder nicht.

15. Verfahren nach Anspruch 13 oder 14, bei dem ein Waschprozess auf die Kapillare angewendet wird, falls detektiert wird, dass eine Substanz an der Kapillare haftet.

## Revendications

1. Dispositif de micro-injection comprenant :
un élément de support définissant un espace intérieur recevant une pression de décharge ;
un capillaire relié à l'élément de support, le capillaire déchargeant un premier fluide d'une extrémité de pointe du capillaire en réponse à la pression de décharge appliquée depuis l'espace intérieur de l'élément de support ; et
un élément de tube relié au capillaire, l'élément de tube définissant un passage d'écoulement pour un second fluide dirigé vers l'extrémité de pointe du capillaire.

2. Dispositif de micro-injection selon la revendication 1, dans lequel le passage d'écoulement s'étend d'une extrémité de base du capillaire jusqu'à l'extrémité de pointe du capillaire en parallèle avec un axe longitudinal du capillaire.

3. Dispositif de micro-injection selon la revendication 1 ou 2, dans lequel le capillaire est placé dans un espace intérieur défini dans l'élément de tube, le capillaire ayant l'extrémité de pointe faisant saillie depuis un orifice de sortie défini à une extrémité de l'élément de tube.

4. Dispositif de micro-injection selon l'une quelconque des revendications précédentes, dans lequel l'élément de tube est formé coaxialement avec le capillaire.

5. Dispositif de micro-injection selon l'une quelconque des revendications précédentes, comprenant en outre :
un mécanisme d'entraînement permettant un mouvement vers l'arrière du capillaire le long de l'axe longitudinal du capillaire ; et
un mécanisme d'alimentation en fluide permettant l'alimentation du second fluide vers le passage d'écoulement pendant le mouvement vers l'arrière du capillaire.

6. Dispositif de micro-injection selon la revendication 5, dans lequel le mécanisme d'alimentation en fluide permet l'alimentation du second fluide vers le passage d'écoulement pendant un mouvement vers l'avant du capillaire.

7. Dispositif de micro-injection selon l'une quelconque des revendications précédentes, comprenant en outre :
un mécanisme d'entraînement permettant un mouvement vers l'avant du capillaire le long d'un axe longitudinal du capillaire ; et
un mécanisme d'alimentation en fluide permettant l'alimentation du second fluide vers le passage d'écoulement pendant le mouvement vers l'avant du capillaire.

8. Dispositif de micro-injection selon l'une quelconque des revendications précédentes, comprenant en outre :
un dispositif de capture d'image utilisé pour capturer une image de l'extrémité de pointe du capillaire ; et
un mécanisme d'alimentation en fluide permettant l'alimentation en fluide du second fluide vers le passage d'écoulement quand une adhésion d'une substance est détectée d'après l'image capturée en utilisant le dispositif de capture d'image.

9. Dispositif de micro-injection selon la revendication 8, comprenant en outre :
un récipient relié à l'élément de tube pour garder un dissolvant pour dissoudre la substance ; et
un récipient relié à l'élément de tube pour contenir un neutralisateur pour neutraliser le dissolvant.

10. Procédé d'injection d'un fluide, comprenant :
d'injecter un premier fluide depuis une extrémité de pointe d'un capillaire dans un objet capturé dans une solution en une position prédéterminée ;
de faire que l'extrémité de pointe du capillaire se retire après que le premier fluide a été injecté ; et
de décharger un second fluide depuis un orifice de sortie quand le capillaire se retire, le second fluide étant dirigé vers l'extrémité de pointe du capillaire.

11. Procédé selon la revendication 10, comprenant en outre :
de déplacer l'extrémité de pointe du capillaire vers l'objet ; et
de décharger le second fluide depuis l'orifice de sortie quand le capillaire se déplace vers l'avant, le second fluide étant dirigé vers l'extrémité de pointe du capillaire.

12. Procédé selon la revendication 10 ou 11, comprenant en outre :
de détecter si ou non une partie adhérente adhère à l'extrémité de pointe du capillaire ;
de régler l'extrémité de pointe du capillaire dans une position opposée à un récipient prédéterminé quand l'adhésion de la partie adhérente a été détectée ;
de décharger un dissolvant de l'orifice de sortie vers l'extrémité de pointe du capillaire pour dissoudre la partie adhérente ; et
de décharger un neutralisateur depuis l'orifice de sortie vers l'extrémité de pointe du capillaire pour neutraliser le dissolvant.

13. Procédé de fabrication d'un corps minuscule, comprenant :
de déplacer un capillaire vers l'avant vers un corps minute pour injecter une substance dans le corps minuscule, un fluide étant déchargé depuis une position adjacente au capillaire vers le corps minuscule ;
d'arrêter la décharge du fluide quand le capillaire est enfoncé dans le corps minuscule ;
d'injecter la substance dans le corps minuscule ; et
de déplacer le capillaire vers l'arrière depuis le corps minuscule, le fluide étant déchargé depuis une position adjacente au capillaire vers le corps minuscule.

14. Procédé selon la revendication 13, dans lequel une image du capillaire est capturée quand la substance est injectée dans le corps minuscule, l'image capturée étant utilisée pour détecter si ou non une substance adhère ou non au capillaire.

15. Procédé selon la revendication 13 ou 14, dans lequel un procédé de lavage est appliqué au capillaire s'il est détecté qu'une substance adhère au capillaire.
